# EUROPEAN PATENT APPLICATION

(11) **EP 4 606 813 A1**
(43) Date of publication of application: **27.08.2025**
(21) Application number: 23879088.5
(22) Date of filing: 17.10.2023
(51) Int. Cl.: C07K 5/062, C07D 257/04, C07D 229/02

(54) **DRUG INTERMEDIATE AND PREPARATION METHOD THEREFOR**

(30) Priority: 18.10.2022 CN 202211275679
(71) Applicant: Fujian Akeylink Biotechnology Co., Ltd., Ningde, Fujian 355300 (CN)
(72) Inventor: ZHANG, Jian, Shanghai 200131 (CN); LIN, Feng, Shanghai 200131 (CN); ZOU, Xiaodong, Shanghai 200131 (CN); WANG, Yong, Shanghai 200131 (CN); GUO, Hui, Shanghai 200131 (CN); XIAO, Fusheng, Shanghai 200131 (CN); HE, Haiying, Shanghai 200131 (CN)
(74) Representative: Murgitroyd & Company
(86) International application number: PCT/CN2023/124877
(87) International publication number: WO 2024/083099

(57) **Abstract**

Disclosed in the present invention are a drug intermediate and a preparation method therefor, and specifically disclosed are a compound as represented by formula (I) and a preparation method therefor.

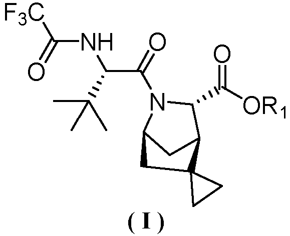

## Description

The present application claims priority of the Chinese Patent Application No. CN 2022112756796 filed on October 18, 2022, the contents of which are incorporated herein by reference in their entireties.

### TECHNICAL FIELD

The present disclosure relates to a pharmaceutical intermediate and a preparation method therefor, specifically to a compound of formula (I) and a preparation method therefor.

### BACKGROUND

2022 is the third year of mankind's fight against the COVID-19 pandemic. The cumulative number of confirmed cases worldwide has exceeded 600 million, and the cumulative number of deaths has exceeded 6 million. About 70 countries have more than 1 million confirmed cases of COVID-19. The epidemiological transmission model, spread, and extent of this pandemic have far exceeded that of the H1N1 influenza Ain 2009. SARS-CoV-2 is a positive-sense single-stranded RNA virus with high homology to SARS-CoV and MERS-CoV. After the virus is infected into a host cell, with the help of the host cell, the genetic material RNA of the virus firstly translates and expresses two polyprotein precursors (pp1a and pp1ab), and the polyprotein precursors are subjected to intramolecular cleavage under the action of 3CL protease and PL protease to generate multiple non-structural proteins. Since 3CL protease is responsible for the cleavage of at least 11 sites, it is also called main protease (Mpro). Non-structural proteins are involved in the generation of viral subgene RNA and four structural proteins (E protein, M protein, S protein, and N protein), thereby completing the reproduction and release of progeny viruses; 3CL protease is a cysteine protease, and its active form is homodimer. 3CL protease is relatively conserved in coronaviruses, and the substrates of 3CL protease of different coronaviruses have common characteristics; since there is no protease homologous to 3CL protease in human body, 3CL proteinase has become one of the ideal anti-coronavirus targets.

The development of drugs that are effective against the novel coronavirus is urgently needed for clinical use. Patent PCT/CN2022/087511 discovered a 3CL protease inhibitor with good anti-novel coronavirus activity. In order to further improve the accessibility of drugs and be more conducive to scale-up production, it is necessary to develop new methods for obtaining intermediates of this 3CL protease inhibitor to overcome the problems associated with the known method in PCT/CN2022/087511, including issues regarding the commercial availability and price of reagents used, the scalability of large-scale production, the safety of process production, and the overall production cost. The molecular structure of 3CL protease inhibitor is as follows:

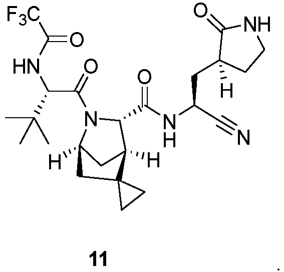

### CONTENT OF THE PRESENT INVENTION

The present disclosure provides a preparation method for a compound of formula (I), 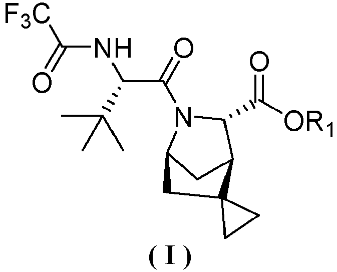 wherein the preparation method comprises a step of synthesizing compound D using compound A as a raw material: 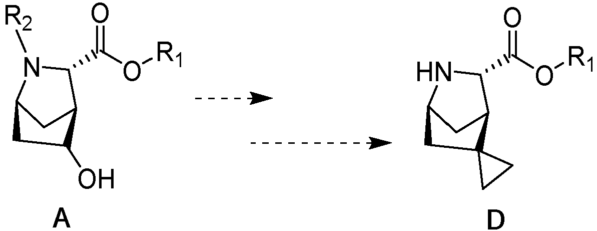 wherein
R₁ is selected from H, C₁₋₄ alkyl, and benzyl;
R₂ is an amino protecting group.

In some embodiments of the present disclosure, the R₁ is selected from H, methyl, ethyl, isopropyl, tert-butyl, and benzyl, preferably methyl.

In some embodiments of the present disclosure, the R₂ is selected from Boc.

In some embodiments of the present disclosure, the preparation method for the compound of formula (I) further comprises a step of synthesizing the compound of formula (I) from compound D.

In some embodiments of the present disclosure, the preparation method for the compound of formula (I) further comprises the following steps:

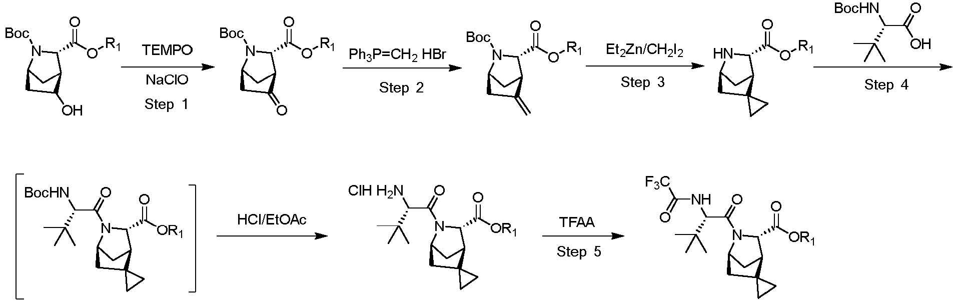

In some embodiments of the present disclosure, the preparation method for the compound of formula (I) further comprises a process of slurrying and purifying a crude product of the resulting compound after the reaction in step 5:
1) adding a mixed solvent of isopropyl acetate and n-heptane to the crude product, wherein the mixed solvent of isopropyl acetate and n-heptane has a ratio of 1:4 to 2:3, preferably 1:3;
2) slurrying at 40°C to 60°C for 1 to 2 hours, preferably at 50°C;
3) cooling to 10°C to 20°C;
4) filtering.

The present disclosure further provides an intermediate of the following formula, a hydrochloride thereof, or a sulfate thereof,

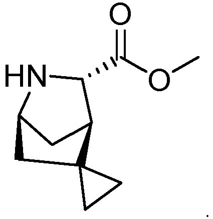

### Definition and description:

Unless otherwise specified, the following terms and phrases when used herein have the following meanings. A specific phrase or term should not be considered indefinite or unclear in the absence of a particular definition, but should be understood in the ordinary sense. When a trade name appears herein, it is intended to refer to its corresponding commodity or active ingredient thereof.

The intermediate compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure. Those skilled in the art can learn from the content of the present disclosure and appropriately change the raw materials, process conditions, and other aspects to achieve corresponding other purposes. The relevant changes do not deviate from the content of the present disclosure. All similar substitutions and modifications are obvious to those skilled in the art and deemed to be within the scope of the present disclosure.

The chemical reactions of the specific embodiments of the present disclosure are completed in a suitable solvent, and the solvent must be appropriate for the chemical changes of the present disclosure and the required reagents and materials thereof. In order to obtain the compounds of the present disclosure, sometimes it is necessary for those skilled in the art to modify or select synthetic methods or reaction schemes on the basis of existing embodiments.

An important consideration in planning any synthetic route in this art is the selection of appropriate protecting groups for reactive functional groups, such as amino groups in the present disclosure.

The term "amino protecting group" refers to a protecting group suitable for preventing the side reactions occurring at the nitrogen of an amino. Representative amino protecting groups include, but are not limited to: formyl; acyl, such as alkanoyl (e.g., acetyl, trichloroacetyl, or trifluoroacetyl); alkoxycarbonyl, such as tert-butoxycarbonyl (Boc); arylmethoxycarbonyl such as benzyloxycarbonyl (Cbz) and 9-fluorenylmethoxycarbonyl (Fmoc); arylmethyl, such as benzyl (Bn), trityl (Tr), 1,1-bis-(4'-methoxyphenyl)methyl; silyl, such as trimethylsilyl (TMS) and tert-butyldimethylsilyl (TBS).

Unless otherwise specified, the term "C₁₋₄ alkyl" refers to a linear or branched saturated hydrocarbon group consisting of 1 to 4 carbon atoms. The C₁₋₄ alkyl includes C₁₋₂, C₁₋₃, C₂₋₃ alkyl, etc.; it can be monovalent (such as methyl), divalent (such as methylene), or multivalent (such as methine). Examples of C₁₋₄ alkyl include, but are not limited to, methyl (Me), ethyl (Et), propyl (including n-propyl and isopropyl), butyl (including n-butyl, isobutyl, s-butyl, and t-butyl), etc.

All solvents used in the present disclosure are commercially available.

The following abbreviations are used in the present disclosure: Me represents methyl; Boc represents tert-butoxycarbonyl; TFAA represents trifluoroacetic anhydride; EtOAc represents ethyl acetate; IBX represents 2-iodoxybenzoic acid; EDCI represents 1-ethyl- (3-dimethylaminopropyl)carbodiimide hydrochloride; HOBt represents 1-hydroxybenzotriazole.

The compounds are named according to the conventional naming methods in the art or by ChemDraw^{®} software, and the commercially available compounds use the supplier catalog names.

### Technical effect

The synthesis process of the intermediate provided by the present disclosure has the beneficial effects of cheap and easily available raw materials, low production costs, elimination of safety hazards in the production process, and more suitable for large-scale production.

### Specifically:

1. Lower cost: In the new route, boc-tert-leucine is used more efficiently, the yield of each step is better, the higher-cost condensation step is moved later, and the material cost is greatly reduced.
2. Cheaper and more readily available reagents: in the original process route, the TEBBE reagent is used in the olefination step; in the new process route, the TEBBE reagent is replaced with a more widely available Wittig reagent.
3. Process safety: The IBX used in the original process route has potential safety hazards during the production process. In the new process route, the IBX is replaced with Tempo reagent, eliminating safety hazards.
4. More suitable for large-scale production: The process conditions involved in the new process route are more conventional, the purification process is simpler, and it is more suitable for large-scale production.

### DETAILED DESCRIPTION OF THE PREFERRED EMBODIMENT

In order to better understand the content of the present disclosure, further description is given below with reference to specific examples, but the specific embodiments are not limitations to the content of the present disclosure.

### Example 1

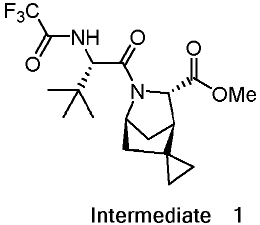

### Synthetic route:

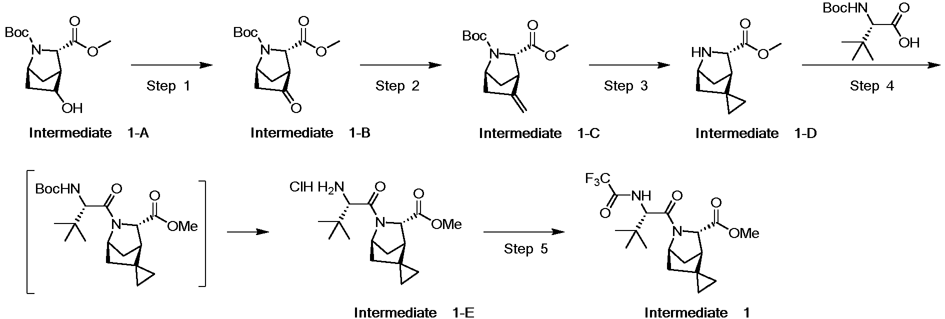

### Step 1: Synthesis of intermediate 1-B

**Intermediate 1-A** (70 g) and 2,2,6,6-tetramethylpiperidinooxy (0.2 g) were dissolved in dichloromethane (700 mL), and the reaction mixture was cooled to 0°C to 5°C. Sodium bicarbonate (33.81 g) and sodium bromide (2.65 g) were dissolved in water (420 mL), and the solution was added to the reaction mixture. Sodium hypochlorite solution (372.12 g, content: 8%) and water (200 mL) were mixed uniformly and added dropwise to the reaction mixture at 0°C to 10°C. The reaction mixture was stirred and reacted for 1 hour, and after the reaction was completed, sodium sulfite (100 g) was dissolved in water (1000 mL), and the solution was added to the reaction mixture to quench for 0.5 hours. The phases were separated, and the organic phase was added with the dichloromethane (1 L) and then extracted again. The organic phases were combined, washed once with the brine (1 L), and concentrated to obtain the **intermediate 1-B** (68 g, yield: 98%). ¹H NMR (400 MHz, CDCl₃) δ ppm major [4.66 (s, 1 H), 4.00 (s, 1H), 3.69 (s, 3 H), 2.94 (s, 1H), 2.37 - 2.12 (m, 3 H), 1.80 (s, 1 H), 1.33(s, 9 H)]. minor [4.53 (s, 1 H), 4.09 (s, 1H), 3.69 (s, 3 H), 2.96 (s, 1H), 2.37 - 2.12 (m, 3 H), 1.77 (s, 1 H), 1.41(s, 9 H)]. MS m/z: 168.0[M-Boc]⁺.

### Step 2: Synthesis of intermediate 1-C

Methyltriphenylphosphonium bromide (103.47 g) was dissolved in toluene (650 mL), and the system was replaced with nitrogen 3 times, cooled to 0°C to 5°C, added with potassium tert-butoxide (29.79 g), and warmed to 20°C to 25°C. The reaction mixture was stirred and reacted for 1 hour, added with **intermediate 1-B** (65 g), and stirred and reacted at 20°C to 25°C for 16 hours. After the reaction was completed, the reaction mixture was added with water (1000 mL), extracted, and the phases were separated. The aqueous phase was added with methyl tert-butyl ether and extracted again. The organic phases were combined, washed with brine (1000 mL), concentrated to dryness under reduced pressure, and the concentrate was purified by column chromatography with petroleum ether-ethyl acetate to obtain the **intermediate 1-C** (33 g, yield: 51%). ¹H NMR (400 MHz, CDCl₃) δ ppm major [5.15 (d, J = 6.0 Hz, 1H), 4.85 (d, J = 6.0 Hz, 1H), 4.45 (s, 1 H), 3.87 (s, 1H), 3.72 (s, 3 H), 3.09 (s, 1H), 2.41 - 2.22 (m, 3 H), 1.84 (s, 1 H), 1.37(s, 9 H)]. minor [5.15 (d, J = 6.0 Hz, 1H), 4.85 (d, J = 6.0 Hz, 1H), 4.31 (s, 1 H), 3.97 (s, 1H), 3.72 (s, 3 H), 3.11 (s, 1H), 2.37 - 2.12 (m, 3 H), 2.06 (s, 1 H), 1.40 (s, 9 H)]. MS m/z: 166.0[M-Boc]⁺.

### Step 3: Synthesis of intermediate 1-D

Chlorobenzene (250 mL) was cooled to -10°C to 0°C and added with diethyl zinc n-hexane solution (1 mol/L, 561 mL). After the dropwise addition, then boron trifluoride diethyl etherate (119.46 g) was added dropwise thereto. After the dropwise addition, the mixture was stirred for 0.5 hours, cooled to -10°C, added dropwise with diiodomethane (300.57 g), and stirred for another 0.5 hours after the dropwise addition. The **intermediate 1-C** (50 g) was dissolved in chlorobenzene (50 mL) and added dropwise to the reaction mixture. After the dropwise addition, the reaction mixture was heated to 35°C to 40°C and reacted for 4 hours. After the reaction was completed, the reaction mixture was cooled to -10°C to -5°C and added dropwise with 20% citric acid aqueous solution (1000 mL). After the dropwise addition, the reaction mixture was added with ethyl acetate (500 mL) and stirred for another 10 minutes, and the phases were separated. The organic phase was washed with 20% citric acid aqueous solution (250 mL), and the aqueous phases were combined, extracted with ethyl acetate (300 mL * 2), and added with a potassium sodium tartrate aqueous solution [potassium sodium tartrate (316 g) + water (750 mL)], and the pH was adjusted to 9 to 10 with ammonia water. Dichloromethane (500 mL) was added thereto, the mixture was extracted, and the phases were separated. The aqueous phase was extracted again with dichloromethane (300 mL), and the organic phases were combined and concentrated under reduced pressure to obtain **intermediate 1-D** (24 g, yield: 70%). ¹H NMR (400 MHz, CDCl₃) δ ppm 3.71 (s, 3H), 3.69 (s, 1H), 3.64 (d, J = 3.6 Hz, 1H), 1.86 (s, 1 H), 1.72 - 1.67 (m, 2 H), 1.52 - 1.47 (m, 2 H), 0.70 - 0.59 (m, 3 H), 0.40 - 0.38 (m, 1 H). MS m/z: 182.1[M+H]⁺.

### Step 4: Synthesis of intermediate 1-E

**Intermediate 1-D** (137 g) and (S)-N-Boc-tert-leucine (192.32 g) were dissolved in a mixture of acetonitrile (1370 mL) and N,N-dimethylformamide (137 mL), then 1-ethyl-(3-dimethylaminopropyl)carbodiimide hydrochloride (159.41 g), N-methylmorpholine (152.93 g), and 1-hydroxybenzotriazole (102.14 g) were added thereto at 25°C, and the reaction mixture reacted for 16 hours. After the reaction was completed, the reaction mixture was concentrated under reduced pressure to remove most of the acetonitrile, added with ethyl acetate (1000 mL) and water (1000 mL), extracted, and the phases were separated. The organic phase was washed with 10% citric acid (500 mL * 2), saturated sodium bicarbonate (500 mL), and saturated brine (500 mL), and concentrated to dryness under reduced pressure. The concentrate was added with hydrochloric acid ethyl acetate solution (4 mol/L, 1.89 L) and methanol (200 mL) at 10 to 15°C. After the addition, the reaction mixture was stirred and reacted at 20°C to 25°C for 0.5 hours. After the reaction was completed, the reaction mixture was concentrated to remove 1 L of solvent, stirred at 25°C for 0.5 hours, and filtered. The filter cake was added with ethyl acetate (200 mL) for washing, and **intermediate 1-E** (212 g, yield: 85%) was obtained after removing residue from the filter cake. ¹H NMR (400 MHz, CDCl₃) δ ppm 8.49 (s, 3H), 4.51 (s, 1 H), 4.42 (s, 1H), 4.03 (d, J = 4.8 Hz, 1H), 3.70 (s, 3 H), 2.26 (d, J = 10.8 Hz, 1H), 2.07 (s, 1H), 1.97 (s, 1H), 1.90 (d, J = 10.0 Hz, 1H), 1.90 (d, J = 10.0 Hz, 1H), 1.80 (d, J = 12.4 Hz, 1H), 1.23 (s, 9 H), 0.81 - 0.64 (m, 3 H), 0.48 - 0.46 (m, 1 H). MS m/z: 294.9[M+H]⁺.

### Step 5: Synthesis of intermediate 1

**Intermediate 1-E** (211.59 g) was dissolved in dichloromethane (1300 mL), then trifluoroacetic anhydride (201.48 g) was added thereto at 10°C to 15°C, and then triethylamine (64.71 g) was added thereto. After the addition, the reaction mixture reacted at 20°C to 25°C for 1 hour. After the reaction was completed, the reaction mixture was cooled to 10°C to 15°C and added dropwise with water (500 mL), and the phases were separated. The organic phase was washed with 10% citric acid aqueous solution (500 mL), saturated sodium bicarbonate aqueous solution (500 mL), and saturated brine (500 mL), concentrated to dryness to obtain the crude product of intermediate 1. The crude product was added with isopropyl acetate (50 mL) and n-heptane (500 mL), and the mixture was slurried at 50°C for 1 to 2 hours, cooled to 10°C to 20°C, and filtered to obtain **intermediate** 1 (212 g, yield: 85%). ¹H NMR (400 MHz, CDCl₃) δ ppm 8.49 (d, J = 8.8 Hz, 1H), 4.68 (d, J = 9.6 Hz, 1H), 4.59 (s, 1 H), 4.37 (s, 1H), 3.72 (s, 3 H), 2.10 (d, J = 10.0 Hz, 1H), 1.99 (s, 1H), 1.92 - 1.86 (m, 2 H), 1.59 (dd, J = 12.4 Hz, 2.4Hz, 1H), 1.10 (s, 9 H), 0.75 - 0.63 (m, 3 H), 0.49 - 0.47 (m, 1 H). MS m/z: 391.1 [M+H]⁺.

### Example 2: Synthesis of intermediate 1-A

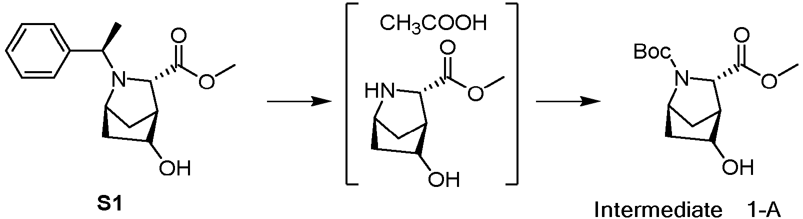

Under nitrogen atmosphere, S1 (39 g, 141.6 mmol, 1 eq) was put into a 1000 mL round-bottomed flask, and 160 g of methanol and acetic acid (10.2 g, 170 mmol, 1.2 eq) were added thereto, stirred, and dissolved; 4.0 g of 10% palladium on carbon was added thereto, the hydrogen pressure was set to 0.3 MPa to 0.5 MPa, the temperature was set to 10°C to 20°C, and the reaction mixture was stirred for 8 hours to 10 hours with the temperature maintained; the central control TLC plate showed that the raw material points disappeared; filtered; the filtrate was transferred to a 1000 mL round-bottomed flask, and added dropwise with triethylamine at 5°C to 5°C, added dropwise with di-tert-butyl dicarbonate at 0°C to 10°C, and the reaction mixture was warmed to 15°C to 20°C after the dropwise addition, and stirred for 2 hours to 2.5 hours with the temperature maintained, and the central control TLC plate showed that the raw material points disappeared; the reaction mixture was concentrated under reduced pressure, and added with 180 g of ethyl acetate and 100 g of drinking water for extracting and washing, and the phases were separated; the aqueous phase was re-extracted with ethyl acetate; the organic phases were combined and washed sequentially with 12% sodium bicarbonate aqueous solution and 30% sodium chloride aqueous solution; the organic phase was dried over anhydrous sodium sulfate for 2 hours, filtered; the filtrate was concentrated to dryness under reduced pressure. After concentration, 80.4 g of n-heptane was added thereto, slurried at room temperature for 2 to 3 hours, and filtered. The filter cake was collected and dried under vacuum at 40°C to 45°C to obtain the target product intermediate 1-A (33.0 g, yield: 85.9%). ¹H NMR (400 MHz, DMSO-d6, 298 K, δ in ppm): 5.03 (m, 1H, CH), 3.94 (m, 1H, CH), 3.71 (s, 9H, C(CH₃)₃), 3.64 (d, 3H, CH₃), 3.60 (d, 1H, CH), 3.34 (m, 2H, CH₂), 2.41 (m, 1H, OH), 1.86 (m, 1H, CH), 1.63 (m, 2H, CH₂).

## Claims

1. A preparation method for a compound of formula (I), 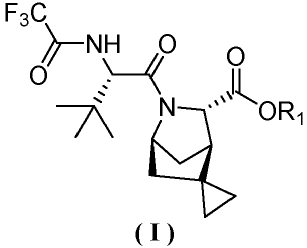 wherein the preparation method comprises a step of synthesizing compound D using compound A as a raw material: 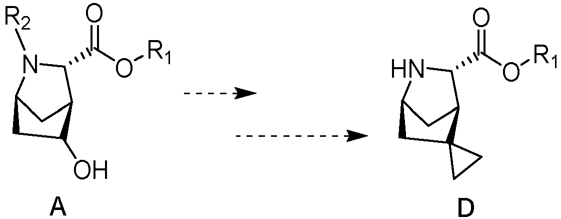 wherein
R₁ is selected from H, C₁₋₄ alkyl, and benzyl;
R₂ is an amino protecting group.

2. The preparation method for the compound of formula (I) according to claim 1, wherein R₁ is selected from H, methyl, ethyl, isopropyl, tert-butyl, and benzyl, preferably methyl.

3. The preparation method for the compound of formula (I) according to claim 1, wherein R₂ is selected from Boc.

4. The preparation method for the compound of formula (I) according to claim 1, wherein the preparation method further comprises a step of synthesizing the compound of formula (I) from compound D.

5. The preparation method for the compound of formula (I) according to claim 1, wherein the preparation method comprises the following steps:

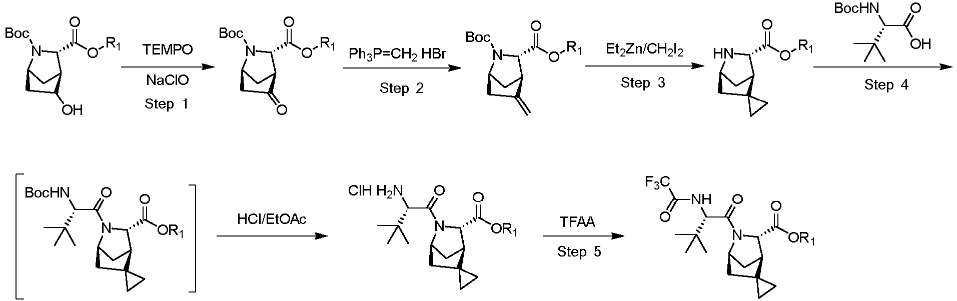

6. The preparation method for the compound of formula (I) according to claim 5, wherein the preparation method further comprises a process of slurrying and purifying a crude product of the resulting compound after the reaction in step 5:
1) adding a mixed solvent of isopropyl acetate and n-heptane to the crude product, wherein the mixed solvent of isopropyl acetate and n-heptane has a ratio of 1:4 to 2:3, preferably 1:3;
2) slurrying at 40°C to 60°C for 1 to 2 hours, preferably at 50°C;
3) cooling to 10°C to 20°C;
4) filtering.

7. An intermediate of the following formula, a hydrochloride thereof, or a sulfate thereof,
